(19) Europäisches Patentamt
European Patent Office
Office européen des brevets

(11) Veröffentlichungsnummer : **0 173 186**
**B1**

# (12) EUROPÄISCHE PATENTSCHRIFT

(45) Veröffentlichungstag der Patentschrift : **21.12.88**

(21) Anmeldenummer : **85110274.9**

(22) Anmeldetag : **16.08.85**

(51) Int. Cl.⁴ : **C 07 D311/32**, C 07 D407/04, C 07 D407/06, C 07 D407/14, A 61 K 31/35, A 61 K 31/36

## (54) Flavanonderivate.

(30) Priorität : **28.08.84 DE 3431534**

(43) Veröffentlichungstag der Anmeldung : **05.03.86 Patentblatt 86/10**

(45) Bekanntmachung des Hinweises auf die Patenterteilung : **21.12.88 Patentblatt 88/51**

(84) Benannte Vertragsstaaten : **AT BE CH DE FR GB IT LI NL SE**

(56) Entgegenhaltungen :
**GB-A- 2 145 718**
**US-A- 3 678 044**
**US-A- 4 241 069**
**Chem. Abstr. 57, 12418f (1962)**

(73) Patentinhaber : **Merck Patent Gesellschaft mit beschränkter Haftung**
**Frankfurter Strasse 250**
**D-6100 Darmstadt (DE)**

(72) Erfinder : **Gericke, Rolf, Dr.**
**Mozartstrasse 19**
**D-6104 Seeheim (DE)**
Erfinder : **Wahlig, Helmut, Dr.**
**Roemheldweg 16**
**D-6100 Darmstadt (DE)**
Erfinder : **Dingeldein, Elvira, Dr.**
**Am Spitzenpfad 9**
**D-6072 Dreieich (DE)**

EP 0 173 186 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue Flavanonderivate der Formel I

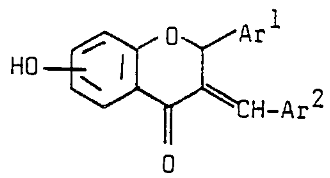

(I)

worin $Ar^1$ und $Ar^2$ jeweils unsubstituiertes oder ein- bis dreifach durch OH, Alkyl, Alkoxy, Acylamino, Halogen, COOAlkyl und/oder $NO_2$ und/oder durch eine Methylendioxygruppe substituiertes Phenyl bedeuten und die Alkyl-, Alkoxy- und Acylgruppen jeweils 1-7 C-Atome haben,
worin jedoch die HO-Gruppe nur dann in 6-Stellung steht, wenn mindestens einer der Reste $Ar^1$ bzw. $Ar^2$ substituiertes Phenyl bedeutet,
sowie ihre Phosphorsäureester (« I-Phosphate ») und die Salze dieser Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I sowie ihre Phosphorsäureester und die Salze dieser Verbindungen wertvolle pharmakologische Eigenschaften besitzen. So zeigen sie beispielsweise antiallergische Wirkungen, die sich z. B. bei intravenöser Gabe an Ratten nachweisen lassen. Konventionelle Methoden zum Nachweis dieser Wirkung sind z. B. in der Literatur beschrieben, die in der EP-A-0056 475 zitiert ist.

Insbesondere sind jedoch bestimmte schwerlösliche Salze der I-Phosphate von Bedeutung, nämlich diejenigen, die von Aminoglykosidantibiotika hergeleitet sind. Diese Salze zeigen ebenfalls antibiotische Wirkungen, zeichnen sich aber durch eine langsamere Freisetzung aus.

6-Hydroxy-3-benzyliden-flavanon (entsprechend Formel I, $Ar^1$ = $Ar^2$ = Phenyl, OH in 6-Stellung) ist in Chem. Abstr. 57, 12418f (1962), der Formel I ähnliche 3-Aryliden-flavanone sind in der US-A-3 678 044 beschrieben ; pharmakologische Eigenschaften dieser Verbindungen sind dort jeweils nicht angegeben.

Weiterhin findet sich in der älteren EP-A-0 139 614 eine Formel

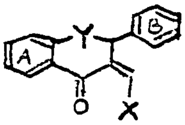

worin X u. a. auch einen unsubstituierten oder substituierten Kohlenwasserstoffrest und Y Sauerstoff bedeuten kann und worin die Ringe A und B substituiert sein können. Ausführungsformen, die unter den vorliegenden Anspruchsgegenstand fallen, sind dort jedoch nicht erwähnt.

In der DE-OS 32 06 725 sind Flavanoidphosphate von Aminoglykosidantibiotika mit ähnlichen Eigenschaften erwähnt. Jedoch finden sich an keiner Stelle dieser DE-OS Hinweise auf die besonders vorteilhaften Salze mit den vorliegenden I-Phosphaten, die sich durch hervorragende Stabilität und günstige Freisetzungskinetik auszeichnen. Außerdem enthalten sie eine Säurekomponente, die exakter definierbar ist als das dort beschriebene, als besonders bevorzugt angegebene Hesperidinphosphat.

Die Verbindungen der Formel I, ihre Phosphate und die Salze dieser Verbindungen können daher als Arzneimittelwirkstoffe in der Human- und Veterinärmedizin verwendet werden. Die Flavanone I selbst sowie die I-Phosphate dienen außerdem als Zwischenprodukte, insbesondere zur Herstellung der genannten Salze.

In der Formel I sind die Reste $Ar^1$ und $Ar^2$ bevorzugt gleich ; sie können jedoch auch voneinander verschieden sein. Bevorzugt bedeuten sie einfach, vorzugsweise in p-Stellung, aber auch in o- oder m-Stellung substituierte Phenylgruppen ; sie können aber auch für unsubstituierte sowie für zweifach, vorzugsweise in 3,4-Stellung, aber auch in 2,3-, 2,4-, 2,5-, 2,6- oder 3,5-Stellung, oder dreifach, vorzugsweise in 3,4,5-Stellung, aber auch in 2,3,4-, 2,3,5-, 2,3,6-, 2,4,5-, 2,4,6-Stellung substituierte Phenylgruppen stehen.

Die Alkyl-, Alkoxy- und Acylgruppen in den Resten $Ar^1$ und $Ar^2$ enthalten jeweils 1-7, vorzugsweise jeweils 1-4, insbesondere 1 oder 2 C-Atome.

Als Alkylgruppen in den Resten $Ar^1$ und $Ar^2$ sind Methyl und Ethyl, weiterhin Propyl, Isopropyl, Butyl, Isobutyl, sek.-Butyl, tert.-Butyl, ferner Pentyl, Isopentyl (= 3-Methylbutyl), Hexyl, Isohexyl (= 4-Methylpentyl) und Heptyl besonders bevorzugt, als Alkoxygruppen Methoxy und Ethoxy, weiterhin Propoxy, Isopropoxy, Butoxy, Isobutoxy, sek.-Butoxy, tert.-Butoxy, ferner Pentoxy, Isopentoxy ( = 3-Methylbutoxy), Hexoxy, Isohexoxy (= 4-Methylpentoxy) und Heptoxy, als Acylaminogruppen Formamido und Acetamido, weiterhin Propionamido, Butyramido, Isobutyramido, ferner Valeramido, Isovaleramido, Trimethylacetamido, Hexanamido, tert.-Butylacetamido und Heptanamido. Halogen steht vorzugsweise für Cl, aber auch

für F, Br oder J, COOAlkyl vorzugsweise für Methoxycarbonyl oder Ethoxycarbonyl, weiterhin für Propoxycarbonyl, Isopropoxycarbonyl, Butoxycarbonyl, Isobutoxycarbonyl, ferner für sek.-Butoxycarbonyl, tert. -Butoxycarbonyl, Pentoxycarbonyl, Hexoxycarbonyl oder Heptoxycarbonyl.

Im einzelnen sind $Ar^1$ und/oder $Ar^2$ bevorzugt p-Methoxyphenyl, weiterhin Phenyl, o-, m- oder p-Hydroxyphenyl, o-, m- oder p-Tolyl, o-, m- oder p-Ethylphenyl, o-, m- oder p-Propylphenyl, o-, m- oder p-Isopropylphenyl, o- oder m-Methoxyphenyl, o-, m- oder p-Ethoxyphenyl, o-, m- oder p-Formamidophenyl, o-, m- oder p-Acetamidophenyl, o-, m- oder p-Fluorphenyl, o-, m- oder p-Chlorphenyl, o-, m- oder p-Bromphenyl, o-, m- oder p-Jodphenyl, o-, m- oder p-Methoxycarbonylphenyl, o-, m- oder p-Ethoxycarbonylphenyl, o-, m- oder p-Nitrophenyl, 2,3- oder 3,4-Methylendioxyphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Dichlorphenyl, 3,4,5-Trimethoxyphenyl, 3-Hydroxy-4-methoxyphenyl, 3-Methoxy-4-hydroxyphenyl.

Die HO-Gruppe in Formel I steht vorzugsweise in der 6-Stellung, sie kann aber auch in der 5-, 7- oder 8-Stellung stehen.

Dementsprechend sind Gegenstand der Erfindung insbesondere diejenigen Verbindungen der Formel I sowie ihre Phosphate und die Salze dieser Verbindungen, in denen mindestens einer der genannten Reste eine der vorstehend angegebenen bevorzugten Bedeutungen hat und/oder in einer der als bevorzugt angegebenen Stellungen steht. Einige bevorzugte Gruppen von Verbindungen können durch die folgenden Teilformeln Ia bis Il ausgedrückt werden, die der Formel I entsprechen und worin die nicht näher bezeichneten Reste die bei der Formel I angegebenen Bedeutungen haben, worin jedoch

in Ia $Ar^1$ Phenyl oder einfach durch OH, Alkyl, Alkoxy oder Acylamino mit jeweils 1-4 C-Atomen, F, Cl, Br, J, COOAlkyl, worin die Alkylgruppe 1-4 C-Atome enthält, $NO_2$ oder Methylendioxy oder zweifach durch Alkoxy mit 1-4 C-Atomen oder Cl oder dreifach durch Alkoxy substituiertes Phenyl bedeutet ;

in Ib $Ar^1$ Phenyl, Isopropylphenyl, Methoxyphenyl, Acetamidophenyl, Chlorphenyl, Dichlorphenyl oder Trimethoxyphenyl bedeutet ;

in Ic $Ar^1$ Phenyl oder Methoxyphenyl bedeutet ;

in Id $Ar^1$ p-Methoxyphenyl bedeutet ;

in Ie $Ar^2$ Phenyl oder einfach durch OH, Alkyl, Alkoxy oder Acylamino mit jeweils 1-4 C-Atomen, F, Cl, Br, J, COOAlkyl, worin die Alkylgruppe 1-4 C-Atome enthält, $NO_2$ oder Methylendioxy oder zweifach durch Alkoxy mit 1-4 C-Atomen oder Cl oder dreifach durch Alkoxy substituiertes Phenyl bedeutet ;

in If $Ar^2$ Phenyl, Isopropylphenyl, Methoxyphenyl, Acetamidophenyl, Chlorphenyl, Dichlorphenyl oder Trimethoxyphenyl bedeutet ;

in Ig $Ar^2$ Phenyl oder Methoxyphenyl bedeutet ;

in Ih $Ar^2$ p-Methoxyphenyl bedeutet ;

in Ii $Ar^1$ und $Ar^2$ jeweils Phenyl oder einfach durch OH, Alkyl, Alkoxy oder Acylamino mit jeweils 1-4 C-Atomen, F, Cl, Br, J, COOAlkyl, worin die Alkylgruppe 1-4 C-Atome enthält, $NO_2$ oder Methylendioxy oder zweifach durch Alkoxy mit 1-4 C-Atomen oder Cl oder dreifach durch Alkoxy substituiertes Phenyl bedeuten ;

in Ij $Ar^1$ und $Ar^2$ jeweils Phenyl, Isopropylphenyl, Methoxyphenyl, Acetamidophenyl, Chlorphenyl, Dichlorphenyl oder Trimethoxyphenyl bedeuten ;

in Ik $Ar^1$ und $Ar^2$ jeweils Phenyl oder Methoxyphenyl ;

in Il $Ar^1$ und $Ar^2$ jeweils p-Methoxyphenyl bedeuten.

Besonders bevorzugt sind ferner Verbindungen der Formeln Iaa bis Ika, die den Formeln Ia bis Ik entsprechen, worin jedoch $Ar^1 = Ar^2$ ist, weiterhin Verbindungen der Formeln Iab bis Ilb sowie Iaab bis Ikab, die den Formeln Ia bis Il sowie Iaa bis Ika entsprechen, worin jedoch die HO-Gruppe in 6-Stellung des Flavanonsystems steht.

Besonders bevorzugt sind ferner die Phosphate der Verbindungen der Formeln Ia bis Il, Iaa bis Ika, Iab bis Ilb und Iaab bis Ikab sowie die Salze dieser Verbindungen, insbesondere die Salze mit Aminoglykosidantibiotika.

Die Verbindungen der Formel I, ihre Phosphate und die Salze dieser Verbindungen können in cis- und in trans-Konfiguration vorliegen. Bevorzugt ist die trans-Konfiguration. Wenn nachstehend nichts anderes angegeben ist, handelt es sich bei den angegebenen Substanzen immer um die trans-Isomeren. Aber auch die cis-Isomeren sind in den Formeln der erfindungsgemäßen Verbindungen eingeschlossen ; sie können durch Bestrahlung aus den trans-Isomeren erhalten werden (Methode vgl. J. org. Chem. 35. 2286 (1970)). Die Formeln der erfindungsgemäßen Verbindungen umschließen auch Isomerengemische.

Gegenstand der Erfindung ist ferner ein Verfahren zur Herstellung von Verbindungen nach Anspruch 1, dadurch gekennzeichnet, daß man ein Flavanon der Formel II

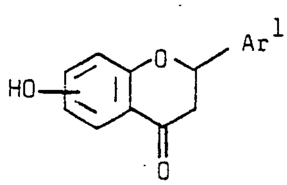

(II)

worin $Ar^1$ die bei Formel I angegebene Bedeutung hat mit einem Aldehyd der Formel III

$$O = CH—Ar^2 \qquad (III)$$

worin $Ar^2$ die bei Formel I angegebene Bedeutung hat umsetzt
oder daß man ein Chalkon der Formel IV

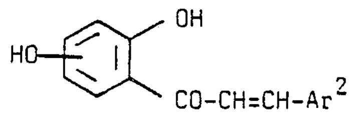

(IV)

worin $Ar^2$ die bei Formel I angegebene Bedeutung hat mit einem Aldehyd der Formel V

$$O = CH—Ar^1 \qquad (V)$$

worin $Ar^1$ die bei Formel I angegebene Bedeutung hat umsetzt
und/oder daß man ein Hydroxyflavanon der Formel I durch Behandeln mit einem phosphorylierenden Mittel in den entsprechenden Phosphorsäureester umwandelt und/oder daß man eine Verbindung der Formel I beziehungsweise ihren Phosphorsäureester durch Behandeln mit einer Base in eines ihrer Salze überführt.

Die Verbindungen der Formel I werden im übrigen nach an sich bekannten Methoden hergestellt, wie sie in der Literatur (z. B. in den Standardwerken wie Houben-Weyl, Methoden der Organischen Chemie, Georg-Thieme-Verlag ; Stuttgart, Organic Reactions, John Wiley & Sons, Inc., New York) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So werden die Verbindungen der Formel I vorzugsweise durch Reaktion von Dihydroxyacetophenonen der Formel VI

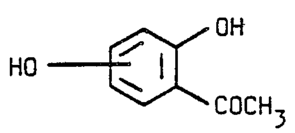

(VI)

mit Aldehyden der Formel III bzw. V hergestellt, wobei die Flavanone II und/oder die Chalkone IV als Zwischenprodukte entstehen, aber nicht isoliert werden. Insbesondere Verbindungen der Formeln I, worin $Ar^1 = Ar^2$ ist, sowie Iaa bis Ika sind auf diese Weise vorteilhaft erhältlich.

Die Ausgangsstoffe der Formeln II bis VI sind größtenteils bekannt (vgl. z. B. US-PS 3 450 717). Sofern sie nicht bekannt sind, können sie nach an sich bekannten Methoden hergestellt werden. So sind die Ausgangsstoffe der Formeln II bzw. IV erhältlich aus Dihydroxyacetophenonen der Formel VI mit Aldehyden der Formeln V bzw. III.

Im einzelnen erfolgen die Umsetzungen von II mit III, IV mit V oder VI mit III oder V in Gegenwart oder Abwesenheit eines inerten Lösungsmittels, z. B. eines Alkohols wie Methanol, Ethanol oder Isopropanol, eines Ethers wie Tetrahydrofuran oder Dioxan eines Esters wie Ethylacetat oder auch in Gemischen dieser Lösungsmittel untereinander und/oder mit Wasser, bei Temperaturen zwischen etwa 0° und etwa 150°, vorzugsweise zwischen 15 und 100°.

Zweckmäßig, wenn auch nicht unbedingt erforderlich, ist die Gegenwart eines sauren oder basischen Katalysators, z. B. einer Mineralsäure wie Chlorwasserstoff, Bromwasserstoff, Schwefelsäure, Phosphorsäure, einer organischen Sulfonsäure wie Methan- oder p-Toluolsulfonsäure, eines Alkalimetallhydroxids wie NaOH oder KOH, eines Amids wie $NaNH_2$, eines Hydrids wie NaH, eines Amins wie Piperidin, Triethylamin oder Pyridin. Ein Überschuß des Katalysators kann auch in einzelnen Fällen als Lösungsmittel dienen.

Die I-Phosphate sind vorzugsweise durch Behandeln der Hydroxyflavanone der Formel I mit phosphorylierenden Mitteln erhältlich. Sofern in den Resten $Ar^1$ und/oder $Ar^2$ des Ausgangsstoffs I eine oder mehrere OH-Gruppen vorhanden sind, können auch diese phosphoryliert werden, so daß man die entsprechenden I-Diphosphate, -Triphosphate usw. erhält.

Phosphorylierende Mittel sind neben der freien Phosphorsäure vor allem Pyrophosphorsäure, Polyphosphorsäure, Phosphorpentoxid, Phosphoroxychlorid, Monochlorphosphorsäure (Gemisch aus Orthophosphorsäure und Phosphoroxychlorid), Phosphorsäuremonobenzylester, Phosphorsäure-dibenzylester-chlorid, Phosphorsäure-mono-(2-cyanäthylester), Phosphorsäuredimorpholid-chlorid.

Die Phosphorylierung des Hydroxyflavanons der Formel I erfolgt in Abwesenheit oder in Gegenwart

eines zusätzlichen Lösungsmittels. Als Lösungsmittel eignen sich vorzugsweise organische Basen wie Pyridin, Triethylamin, Chinolin, Dimethylanilin, Diethylanilin, falls bei der Umsetzung eine Säure, z. B. Chlorwasserstoff, abgespalten wird. Andernfalls oder zusätzlich können inerte organische Lösungsmittel verwendet werden, wie z. B. Diethyl- oder Diisopropylether, Tetrahydrofuran, Dioxan, Chloroform, Methylenchlorid, Trichlorethylen, Dimethylformamid, Dimethylsulfoxid, Benzol, Toluol, Xylol, Tetralin, Acetonitril. Ferner können Gemische der vorstehenden Basen und/oder Lösungsmittel verwendet werden. Es ist auch möglich, die Reaktion in einem Überschuß des phosphorylierenden Mittels ablaufen zu lassen. Die Reaktionstemperaturen liegen zwischen —80 und + 200°, vorzugsweise zwischen —10 und + 100°.

Ganz allgemein können zur Herstellung der I-Phosphate und ihrer Salze die Verfahren verwendet werden, die in Houben-Weyl, l.c., Band XII/2, Seiten 143-210 (1964), beschrieben sind.

Bei der Herstellung der Verbindungen der Formel I und ihrer Phosphorsäureeester können Zwischenprodukte entstehen, die Schutzgruppen enthalten. Diese können hydrolytisch oder hydrogenolytisch entfernt werden. Insbesondere können geschützte Hydroxygruppen durch alkalische oder durch vorsichtige saure Hydrolyse in Freiheit gesetzt werden. Solche geschützten Hydroxygruppen können der Flavanoidkomponente, vorzugsweise aber der Phosphorsäure-Komponente der Veresterungsreaktion entstammen. Hat man beispielsweise mit Phosphorsäure-mono-(2-cyanethylester), Phosphorsäurediphenylester-chlorid oder Phosphorsäure-dimorpholidchlorid verestert, so können die erhaltenen Phosphorsäure-di- bzw. triester bzw. -monoester-diamide mit z. B. Alkali- oder Ammoniumhydroxid-Lösungen, basischen oder sauren Ionenaustauschern zu den gewünschten Flavanoid-phosphorsäureestern gespalten werden.

Hydrogenolytisch können Schutzgruppen, vorzugsweise Benzylgruppen in Phosphorsäureestern, beispielsweise durch katalytische Hydrierung abgespalten werden, vorzugsweise unter milden Bedingungen, etwa mit einem Palladiumkatalysator wie Palladium auf Kohle, Calciumcarbonat oder Strontiumcarbonat sowie bei Raumtemperatur und Normaldruck, wobei man die Hydrierung nach Aufnahme der berechneten Wasserstoffmenge zweckmäßig abbricht.

Die Hydroxyflavanone der Formel I sowie ihre Phosphorsäureester können durch Behandeln mit einer Base in die zugehörigen Salze umgewandelt werden. Als Salze kommen die Phenolate der Hydroxyflavanone I, vor allem aber die Salze der I-Phosphate in Betracht.

Bevorzugt sind physiologisch unbedenkliche Salze. Die Salze werden in der Regel bei Raumtemperatur hergestellt, wobei man als Lösungmittel vornehmlich Wasser, Alkohole wie Methanol oder Ethanol, Gemische von Wasser mit Alkoholen oder die zur Salzbildung herangezogenen Basen verwendet. Als Basen sind vorzugsweise geeignet die Hydroxide, Carbonate oder Alkoholate der Alkali- und Erdalkalimetalle sowie die entsprechenden Ammoniumverbindungen, vorzugsweise Natrium-, Kalium-, Calcium- oder Magnesiumhydroxid, Natrium-, Kalium-, Calcium- und Magnesiumcarbonat, Natrium-, Kalium-, Calcium- oder Magnesiumbicarbonat, Natrium-, Kalium-, Calcium- oder Magnesiummethylat, -ethylat, -isopropylat oder -tert.-butylat, ferner Ammoniumhydroxid, -carbonat oder -bicarbonat, sowie substituierte Ammoniumhydroxide, -carbonate oder -bicarbonate.

Insbesondere sind jedoch Salze der I-Phosphate mit Aminoglykosidantibiotika von Bedeutung.

Als Aminoglykosidantibiotika kommen insbesondere diejenigen in Betracht, die eine Desoxystreptamin-Einheit enthalten. Im einzelnen sind besonders bevorzugt Amikacin, Dibekacin, Gentamycin, Neomycine, Paromomycin, Sagamycin, Sisomicin, Streptomycin und Tobramycin, weiterhin sind z. B. bevorzugt Allomycin, Amicetin, Apramycin, Bekanamycin, Betamicin, Butirosin, Destomycin, Everninomycine, Ezomycine, Flambamycin, Fortimycin A und B, Framycetin, Hikizimycin, Homomycin, Hybrimycin, Hygromycin, Kanamycine, Kasugamycin, Lividomycin, Minosaminomycin, Myomycine, Netilmicin, Parvulomycin, Puromycin A, Ribostamycin, Rimocidin, Ristomycin, Ristosamin, Seldomycine, Sorbistin, Spectinomycin, Streptothricin, Tunicamycin und Verdamycin sowie deren Epimeren und Derivate, soweit sie basisch sind.

Da einige dieser Antibiotika, z. B. Gentamycin, bekanntlich keine einheitlichen Substanzen sind, sondern Gemische (Gentamycin z. B. ein Gemisch der Verbindungen Gentamycin C 1, Gentamycin C 2 und Gentamycin C 1a) darstellen, sind auch die Salze der I-Phosphate in einzelnen Fällen keine einheitlichen Substanzen, sondern Gemische. Da viele der genannten Antibiotika, z. B. alle Gentamycine, mehrere basische Stickstoffatome enthalten und da andererseits die I-Phosphate mehrbasige Säuren sind, ist es außerdem möglich, daß sich saure, neutrale und/oder basische Salze bilden. Alle diese möglichen Salze und ihre Gemische untereinander sind in der Definition « Salze der Phosphorsäureester von Flavanonen der Formel I mit Aminoglykosidantibiotika » eingeschlossen.

Bevorzugt sind die neutralen Salze und diese enthaltenden Gemische ; im Fall der Gentamycinsalze sind z. B. besonders bevorzugt die Salze (Gemische) aus 1 Mol Gentamycin und 3 bis 5, insbesondere etwa 4 Mol I-Phosphat, insbesondere das Salz aus 1 Mol Gentamycin und etwa 4 Mol 3-p-Methoxybenzyliden-6-hydroxy-4'-methoxy-flavanon-6-phosphat ; dieses Salz wird nachstehend als « G » bezeichnet. Die sauren Protonen des Salzes, die nicht durch die Aminogruppen des Antibiotikums neutralisiert sind, können in freier (saurer) Form vorliegen oder mit Hilfe von Natriumionen oder anderen physiologisch unbedenklichen Ionen neutralisiert werden.

Die Salze der I-Phosphate mit den Aminoglykosidantibiotika werden in an sich bekannter Weise hergestellt, etwa durch Zusammengeben einer wässerigen Lösung des wasserlöslichen Salzes des

Antibiotikums (z. B. Gentamycinsulfat) mit einer wässerigen Lösung des I-Phosphats oder eines seiner wasserlöslichen Salze (z. B. des Dinatriumsalzes), zweckmäßig unter Rühren bei Raumtemperatur bei pH-Werten zwischen 4 und 8. Zur Verbesserung der Löslichkeit kann auch ein organisches Lösungsmittel zugesetzt werden, z. B. ein Alkohol wie Ethanol. Die gebildeten Antibiotika-Salze sind in Wasser schwer löslich und können durch Abfiltrieren, Waschen mit Wasser und Trocknen erhalten werden.

Die Verbindungen der Formel I bzw. ihre Phosphate können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel, insbesondere für die I-Phosphate eignen sich z. B. optisch aktive Basen, z. B. Chinin, Chinidin, Cinchonin, Cinchonidin, Brucin, Dehydroabietylamin, Strychnin, Morphin, die D- und L-Formen von 1-Phenylethylamin, Fenchylamin, Menthylamin oder von basischen Aminosäuren, z. B. Arginin oder Lysin, oder von deren Estern. Die verschiedenen Formen der Diastereomeren können in an sich bekannter Weise, z. B. durch fraktionierte Kristallisation, getrennt, und die optisch aktiven Verbindungen der Formel I bzw. ihre Phosphate können in an sich bekannter Weise aus den Diastereomeren in Freiheit gesetzt werden.

Gegenstand der Erfindung ist ferner die Verwendung der Verbindungen der Formel I, ihrer Phosphorsäureester sowie der Salze dieser Verbindungen, vor allem der genannten Salze mit Aminoglykosidantibiotika, zur Herstellung pharmazeutischer Zubereitungen, insbesondere auf nicht-chemischem Wege. Hierbei können sie zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner pharmazeutische Zubereitungen, enthaltend mindestens eine Verbindung der Formel I und/oder einen ihrer Phosphorsäureester und/oder ein physiologisch unbedenkliches Salz einer dieser Verbindungen.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, Suspensionen, Emulsionen oder Implantate, für die topische Anwendung Salben, Cremes oder Puder. Implantate, beispielsweise auf der Basis von Silikonkautschuk, Tricalciumphosphat oder Kollagen, die sich z. B. zur Behandlung des infizierten Knochens eignen, sind von besonderer Bedeutung, insbesondere für die genannten Antibiotika-Salze. Aus diesen Implantaten wird das Antibiotikum protrahiert freigesetzt, so daß in der Umgebung des Implantats lang anhaltend wirksame Spiegel des Antibiotikums vorhanden sind. Auch Fibrin-Antibiotikum-Gele, wie sie z. B. in der DE-OS 32 06 725 beschrieben sind, eignen sich zur therapeutischen Anwendung. Die neuen Verbindungen können auch lyophilisiert und die erhaltenen Lyophilisate z. B. zur Herstellung von Injektionspräparaten verwendet werden. Die angegebene Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten ; Zubereitungen, die Antibiotika-Salze der I-Phosphate enthalten, können z. B. zusätzlich leicht lösliche Salze der gleichen oder anderer Antibiotika enthalten, um zusätzlich zu dem Depot-Effekt, der durch die Salze der I-Phosphate bewirkt wird, eine systemische Wirkung zu erzielen.

Gegenstand der Erfindung ist ferner die Verwendung der Verbindungen der Formel I, ihrer Phosphorsäureester und/oder der physiologisch unbedenklichen Salze dieser Verbindungen bei der Bekämpfung von Krankheiten, insbesondere von Allergien und/oder bakteriellen Infektionen, sowie ihre Verwendung bei der therapeutischen Behandlung des menschlichen oder tierischen Körpers.

Dabei werden die erfindungsgemäßen Substanzen in der Regel in Analogie zu bekannten im Handel befindlichen Antiallergika bzw. Bronchospasmolytika/Antiasthmatika, z. B. Cromoglicinsäure und ihren Salzen, oder Antibiotika verabreicht, vorzugsweise in Dosierungen zwischen etwa 5 und 1 000 mg, insbesondere zwischen 10 und 500 mg pro Dosierungseinheit (bei den Antibiotika-Salzen bezogen auf Antibiotikum-Wirkstoff). Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die lokale Applikation ist bevorzugt.

Vor- und nachstehend sind die Temperaturen in °C angegeben. IR = Maxima des Infrarotspektrums in KBr.

Beispiel 1

In eine Lösung von 15,2 g 2,5-Dihydroxyacetophenon und 27,2 g Anisaldehyd in 1 500 ml Ethanol wird 4 Stunden unter Rühren HCl eingeleitet. 3-p-Methoxybenzyliden-6-hydroxy-4'-methoxy-flavanon fällt aus, F. 199-201°. Intermediär entstehen 6-Hydroxy-4'-methoxy-flavanon und 2,5-Dihydroxy-p-methoxy-benzyliden-acetophenon.

Analog sind mit den entsprechenden Aldehyden erhältlich :

3-o-Hydroxybenzyliden-6,2'-dihydroxyflavanon
3-m-Hydroxybenzyliden-6,3'-dihydroxyflavanon
3-p-Hydroxybenzyliden-6,4'-dihydroxyflavanon
3-p-Methylbenzyliden-6-hydroxy-4'-methylflavanon
3-p-Ethylbenzyliden-6-hydroxy-4'-ethylflavanon
3-p-Isopropylbenzyliden-6-hydroxy-4'-isopropylflavanon, F. 178-180°
3-o-Methoxybenzyliden-6-hydroxy-2'-methoxyflavanon
3-m-Methoxybenzyliden-6-hydroxy-3'-methoxyflavanon
3-p-Methoxybenzyliden-5-hydroxy-4'-methoxyflavanon
3-p-Methoxybenzyliden-7-hydroxy-4'-methoxyflavanon
3-p-Methoxybenzyliden-8-hydroxy-4'-methoxyflavanon
3-(3,4-Dimethoxybenzyliden)-6-hydroxy-3',4'-dimethoxyflavanon
3-(3,4,5-Trimethoxybenzyliden)-6-hydroxy-3',4',5'-trimethoxyflavanon, F. 168-170°
3-p-Acetamidobenzyliden-6-hydroxy-4'-acetamidoflavanon, F. 205°
3-p-Fluorbenzyliden-6-hydroxy-4'-fluorflavanon
3-p-Chlorbenzyliden-6-hydroxy-4'-chlorflavanon
3-(3,4-Dichlorbenzyliden)-6-hydroxy-3',4'-dichlorflavanon, F. 228-230°
3-p-Brombenzyliden-6-hydroxy-4'-bromflavanon
3-p-Jodbenzyliden-6-hydroxy-4'-jodflavanon
3-p-Methoxycarbonylbenzyliden-6-hydroxy-4'-methoxycarbonylflavanon (als Lösungsmittel wird Methanol verwendet)
3-p-Ethoxycarbonylbenzyliden-6-hydroxy-4'-ethoxycarbonylflavanon
3-o-Nitrobenzyliden-6-hydroxy-2'-nitroflavanon
3-m-Nitrobenzyliden-6-hydroxy-3'-nitroflavanon
3-p-Nitrobenzyliden-6-hydroxy-4'-nitroflavanon
3-(3,4-Methylendioxybenzyliden)-6-hydroxy-3',4'-methylendioxyflavanon.

Beispiel 2

Man sättigt eine heiße Lösung von 27 g 6-Hydroxy-4'-methoxyflavanon und 13,6 g Anisaldehyd in 1400 ml Ethanol mit HCl, läßt 3 Stunden abkühlen, fällt mit Wasser und erhält 3-p-Methoxybenzyliden-6-hydroxy-4'-methoxyflavanon, F. 199-201°.

Analog sind die in Beispiel 1 angegebenen Verbindungen aus den entsprechenden 6-Hydroxyflavanonen und den entsprechenden Aldehyden erhältlich.

Ferner sind analog erhältlich :

3-Benzyliden-5-hydroxyflavanon
3-Benzyliden-7-hydroxyflavanon
3-Benzyliden-8-hydroxyflavanon
3-p-Methoxybenzyliden-5-hydroxyflavanon
3-p-Methoxybenzyliden-6-hydroxyflavanon
3-p-Methoxybenzyliden-7-hydroxyflavanon
3-p-Methoxybenzyliden-8-hydroxyflavanon
3-Benzyliden-5-hydroxy-4'-methoxyflavanon
3-Benzyliden-6-hydroxy-4'-methoxyflavanon
3-Benzyliden-7-hydroxy-4'-methoxyflavanon
3-Benzyliden-8-hydroxy-4'-methoxyflavanon
3-o-Hydroxybenzyliden-6-hydroxy-4'-methoxyflavanon
3-m-Hydroxybenzyliden-6-hydroxy-4'-methoxyflavanon
3-p-Hydroxybenzyliden-6-hydroxy-4'-methoxyflavanon
3-(2,4-Dihydroxybenzyliden)-6-hydroxy-4'-methoxyflavanon
3-(3-Methoxy-4-hydroxybenzyliden)-6-hydroxy-4'-methoxyflavanon
3-o-Methylbenzyliden-6-hydroxy-4'-methoxyflavanon
3-m-Methylbenzyliden-6-hydroxy-4'-methoxyflavanon
3-p-Methylbenzyliden-6-hydroxy-4'-methoxyflavanon
3-p-Isopropylbenzyliden-6-hydroxy-4'-methoxyflavanon
3-p-Heptylbenzyliden-6-hydroxy-4'-methoxyflavanon

3-o-Methoxybenzyliden-6-hydroxy-4'-methoxyflavanon
3-m-Methoxybenzyliden-6-hydroxy-4'-methoxyflavanon
3-p-Ethoxybenzyliden-6-hydroxy-4'-methoxyflavanon
3-p-Heptoxybenzyliden-6-hydroxy-4'-methoxyflavanon
3-(3,4-Dimethoxybenzyliden)-6-hydroxy-4'-methoxyflavanon
3-(3,4,5-Trimethoxybenzyliden)-6-hydroxy-4'-methoxyflavanon
3-p-Formamidobenzyliden-6-hydroxy-4'-methoxyflavanon
3-o-Acetamidobenzyliden-6-hydroxy-4'-methoxyflavanon
3-m-Acetamidobenzyliden-6-hydroxy-4'-methoxyflavanon
3-p-Acetamidobenzyliden-6-hydroxy-4'-methoxyflavanon
3-p-Propionamidobenzyliden-6-hydroxy-4'-methoxyflavanon
3-p-Butyramidobenzyliden-6-hydroxy-4'-methoxyflavanon
3-p-Heptanamidobenzyliden-6-hydroxy-4'-methoxyflavanon
3-p-Benzamidobenzyliden-6-hydroxy-4'-methoxyflavanon
3-p-Fluorbenzyliden-6-hydroxy-4'-methoxyflavanon
3-p-Chlorbenzyliden-6-hydroxy-4'-methoxyflavanon
3-(3,4-Dichlorbenzyliden)-6-hydroxy-4'-methoxyflavanon
3-p-Brombenzyliden-6-hydroxy-4'-methoxyflavanon
3-p-Jodbenzyliden-6-hydroxy-4'-methoxyflavanon
3-p-Methoxycarbonylbenzyliden-6-hydroxy-4'-methoxyflavanon
3-p-Ethoxycarbonylbenzyliden-6-hydroxy-4'-methoxyflavanon
3-o-Nitrobenzyliden-6-hydroxy-4'-methoxyflavanon
3-m-Nitrobenzyliden-6-hydroxy-4'-methoxyflavanon
3-p-Nitrobenzyliden-6-hydroxy-4'-methoxyflavanon
3-(3,4-Methylendioxybenzyliden)-6-hydroxy-4'-methoxyflavanon.

## Beispiel 3

Ein Gemisch von 2,7 g 2,5-Dihydroxy-p-methoxybenzyliden-acetophenon, 1,36 g Anisaldehyd und 5 ml Piperidin wird 1 Stunde auf 100° erwärmt, abgekühlt und mit Wasser versetzt. 3-p-Methoxybenzyliden-6-hydroxy-4'-methoxyflavanon fällt aus ; F. 199-201°.

Analog sind mit den entsprechenden Aldehyden erhältlich :

3-p-Methoxybenzyliden-6-hydroxyflavanon
3-p-Methoxybenzyliden-6,2'-dihydroxyflavanon
3-p-Methoxybenzyliden-6,3'-dihydroxyflavanon
3-p-Methoxybenzyliden-6,4'-dihydroxyflavanon
3-p-Methoxybenzyliden-6,3',4'-trihydroxyflavanon
3-p-Methoxybenzyliden-6,4'-dihydroxy-3'-methoxyflavanon
3-p-Methoxybenzyliden-6-hydroxy-2'-methylflavanon
3-p-Methoxybenzyliden-6-hydroxy-3'-methylflavanon
3-p-Methoxybenzyliden-6-hydroxy-4'-methylflavanon
3-p-Methoxybenzyliden-6-hydroxy-4'-isopropylflavanon
3-p-Methoxybenzyliden-6-hydroxy-4'-heptylflavanon
3-p-Methoxybenzyliden-6-hydroxy-2'-methoxyflavanon
3-p-Methoxybenzyliden-6-hydroxy-3'-methoxyflavanon
3-p-Methoxybenzyliden-6-hydroxy-4'-ethoxyflavanon
3-p-Methoxybenzyliden-6-hydroxy-4'-heptoxyflavanon
3-p-Methoxybenzyliden-6-hydroxy-3',4'-dimethoxyflavanon
3-p-Methoxybenzyliden-6-hydroxy-3',4',5'-trimethoxyflavanon
3-p-Methoxybenzyliden-6-hydroxy-4'-formamidoflavanon
3-p-Methoxybenzyliden-6-hydroxy-2'-acetamidoflavanon
3-p-Methoxybenzyliden-6-hydroxy-3'-acetamidoflavanon
3-p-Methoxybenzyliden-6-hydroxy-4'-acetamidoflavanon
3-p-Methoxybenzyliden-6-hydroxy-4'-propionamidoflavanon
3-p-Methoxybenzyliden-6-hydroxy-4'-butyramidoflavanon
3-p-Methoxybenzyliden-6-hydroxy-4'-heptanamidoflavanon
3-p-Methoxybenzyliden-6-hydroxy-4'-benzamidoflavanon
3-p-Methoxybenzyliden-6-hydroxy-4'-fluorflavanon
3-p-Methoxybenzyliden-6-hydroxy-4'-chlorflavanon
3-p-Methoxybenzyliden-6-hydroxy-2',4'-dichlorflavanon
3-p-Methoxybenzyliden-6-hydroxy-3',4'-dichlorflavanon
3-p-Methoxybenzyliden-6-hydroxy-4'-bromflavanon
3-p-Methoxybenzyliden-6-hydroxy-4'-jodflavanon
3-p-Methoxybenzyliden-6-hydroxy-4'-methoxycarbonylflavanon

3-p-Methoxybenzyliden-6-hydroxy-4'-ethoxycarbonylflavanon
3-p-Methoxybenzyliden-6-hydroxy-2'-nitroflavanon
3-p-Methoxybenzyliden-6-hydroxy-3'-nitroflavanon
3-p-Methoxybenzyliden-6-hydroxy-4'-nitroflavanon
3-p-Methoxybenzyliden-6-hydroxy-3',4'-methylendioxyflavanon.

Beispiel 4

Eine Lösung von 38,8 g 3-p-Methoxybenzyliden-6-hydroxy-4'-methoxyflavanon in 300 ml Pyridin wird bei 10-15° unter Rühren zu einer Lösung von 100 ml $POCl_3$ in 500 ml Pyridin zugetropft. Man rührt noch 2 Stunden bei 20°, gibt das Gemisch dann in 7 l verdünnte Salzsäure, konzentriert die Lösung, arbeitet mit Wasser und Ethylacetat auf, versetzt den organischen Extrakt erneut mit Wasser, stellt mit NaOH auf pH 6 ein, dampft ein und erhält so das Dinatriumsalz des 3-p-Methoxybenzyliden-6-hydroxy-4'-methoxyflavanon-6-phosphats.

Kernresonanzspektrum (DMSO + $CF_3COOD$) : 3,73 (s), 3,81 (s), 6,7 (s), 6,9-7 (m) und 8,01 (s) ppm (s = Singlett, m = Multiplett).

Analog erhält man :

3-Benzyliden-6-hydroxyflavanon-6-phosphat, F. 90-92°
3-p-Isopropylbenzyliden-6-hydroxy-4'-isopropylflavanon-6-phosphat
3-(3,4,5-Trimethoxybenzyliden)-6-hydroxy-3',4',5'-trimethoxyflavanon-6-phosphat, F. 153-155°
3-p-Acetamidobenzyliden-6-hydroxy-4'-acetamidoflavanon-6-phosphat
3-(3,4-Dichlorbenzyliden)-6-hydroxy-3',4'-dichlorflavanon-6-phosphat, F. 128-130°
3-p-Methoxybenzyliden-6-hydroxy-4'-methoxyflavanon-6-phosphat
3-Benzyliden-6-hydroxy-4'-methoxyflavanon-6-phosphat

sowie die Phosphorsäureester der übrigen in den Beispielen 1 bis 3 genannten 3-Benzylidenflavanone.

Beispiel 5

Eine Lösung von 3,88 g 3-p-Methoxybenzyliden-6-hydroxy-4'-methoxyflavanon in 40 ml Pyridin wird bei — 25° mit 20 ml einer Lösung von 14 g Phosphorsäuredibenzylesterchlorid in Ether versetzt, 1 Stunde bei — 25° gerührt und 16 Stunden bei — 5° stehengelassen. Man gießt auf Eis, säuert mit Salzsäure auf pH 4 an, extrahiert mit Ether und trocknet über Natriumsulfat. Der aus der Etherlösung erhaltene Rückstand wird in 200 ml Methanol gelöst und an 0,3 g 10 %iger Pd-Kohle bei 1 bar und 20° bis zum Stillstand hydriert. Man filtriert den Katalysator ab, dampft ein und erhält 3-p-Methoxybenzyliden-6-hydroxy-4'-methoxyflavanon-6-phosphat.

Beispiel 6

Ein Gemisch von 3,88 g 3-p-Methoxybenzyliden-6-hydroxy-4'-methoxyflavanon, 34 g Phosphorsäuremonobenzylester, 19 g Dimethylformamidchlorid und 150 ml Pyridin wird 2 Stunden bei 20° gerührt, 16 Stunden bei 20° stehengelassen und analog Beispiel 5 aufgearbeitet. Man erhält das gleiche Produkt.

Beispiel 7

Ein Gemisch von 2 ml wasserfreier Phosphorsäure, 17 ml Acetonitril und 5 ml Triethylamin wird auf — 20° abgekühlt, mit 3,6 ml Chlorameisensäurethylester in 4 ml Acetonitril versetzt und 15 Minuten gerührt. Dann gibt man eine Lösung von 3,88 g 3-p-Methoxybenzyliden-6-hydroxy-4'-methoxyflavanon in 12 ml Acetonitril sowie 4,8 ml Triethylamin unter Rühren zu, kocht nach dem Ende der Gasentwicklung noch 1 Stunde, arbeitet analog Beispiel 5 auf und erhält das gleiche Produkt.

Beispiel 8

Eine etherische Lösung von Monochlorphosphorsäure, erhalten durch Einwirkung von 9,4 g $POCl_3$ auf 10,2 g 85 %ige $H_3PO_4$, wird bei — 10° zu einer Lösung von 3,88 g 3-p-Methoxybenzyliden-6-hydroxy-4'-methoxyflavanon in 20 ml Pyridin gegeben. Nach 15stündigem Stehen wird analog Beispiel 5 aufgearbeitet. Man erhält das gleiche Produkt.

Beispiel 9

Eine gemäß Beispiel 4 hergestellte Lösung des Dinatriumsalzes des 3-p-Methoxybenzyliden-6-hydroxy-4'-methoxyflavanon-6-phosphats wird unter Rühren mit einer Lösung von 17,6 g Gentamycinsulfat in 200 ml Wasser versetzt. Das erhaltene Salz der ungefähren Zusammensetzung 1 Gentamycin. 4 3-p-Methoxybenzyliden-6-hydroxy-4'-methoxyflavanon-6-phosphat (« G ») wird abfiltriert und getrocknet. IR : 1615, 1520, 1490, 1270, 1190, 1180, 1040 $cm^{-1}$.

Analog sind die Gentamycinsalze der übrigen in Beispiel 4 genannten Phosphorsäureester erhältlich, z. B. diejenigen von 3-Benzyliden-6-hydroxyflavanon-6-phosphat, IR : 1670, 1620, 1490, 1070, 980, 920 $cm^{-1}$ ;

3-(3,4,5-Trimethoxybenzyliden)-6-hydroxy-3',4',5'-trimethoxyflavanon-6-phosphat, IR : 1590, 1510, 1490, 1250, 1135 $cm^{-1}$.

## Beispiel 10

Analog Beispiel 9 erhält man aus den berechneten Mengen der Sulfate der entsprechenden Antibiotika und dem Dinatriumsalz des 3-p-Methoxybenzyliden-6-hydroxy-4'-methoxyflavanon-6-phosphats folgende Salze dieser Verbindung :

a) mit Neomycin ;
b) mit Paromomycin ;
c) mit Sisomycin ;
d) mit Amikacin ;
e) mit Tobramycin ;
f) mit Dibekacin ;
g) mit Streptomycin.

Die nachstehenden Beispiele betreffen pharmazeutische Zubereitungen, die erfindungsgemäße Verbindungen enthalten :

## Beispiel A : Tabletten

Ein Gemisch von 1 kg Mono-Na-Salz des 3-p-Methoxybenzyliden-6-hydroxy-4'-methoxy-flavanon-6-phosphats, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 50 mg Wirkstoff enthält.

## Beispiel B : Dragees

Analog Beispiel A werden Tabletten gepreßt, die anschließend in üblicher Weise mit einem Überzug aus Saccharose, Kartoffelstärke, Talk, Tragant und Farbstoff überzogen werden.

## Beispiel C : Kapseln

Man füllt 10 kg « G » in üblicher Weise in Hartgelatinekapseln, so daß jede Kapsel Wirkstoff entsprechend 165 mg Gentamycin-Base enthält.

## Beispiel D : Ampullen

Man mikronisiert 1 kg « G » fein, suspendiert in 30 l Sesamöl und füllt in Ampullen ab, die unter sterilen Bedingungen verschlossen werden. jede Ampulle enthält Wirkstoff entsprechend 10 (40, 80, 120) mg Gentamycin-Base.

## Beispiel E : Implantate

Man mischt 1,05 g mikronisiertes « G » (entsprechend 0,2 g Gentamycin) mit 8,5 g Silikonkautschuk-Monomerem (Medical Grade Silastic 382, Dow Corning), gibt 2 Tropfen Polymerisationskatalysator hinzu, mischt erneut und formt zu runden Scheiben von 20 mm Durchmesser und 1 mm Dicke. Jede Scheibe enthält 6 mg Gentamycin-Base.

## Beispiel F : Fibrin-Antibiotikum-Gel

Man löst 4 NIH-Einheiten Thrombin (Handelspräparat) in 1 ml Aprotinin-Calciumchlorid-Lösung (Handelspräparat ; 3000 KIE/ml Aprotinin in 40 mMol/l $CaCl_2$) erwärmt die Lösung auf 37°, gibt eine Menge « G », die 20 mg Gentamycin-Base entspricht, hinzu und mischt mit der gleichen Menge vorher auf 37° erwärmten « Fibrinkleber » (Handelspräparat ; hergestellt durch Kältepräzipitation aus humanem Spender-plasma ; bei —18° oder kälter gelagert ; 1 ml der Lösung enthält durchschnittlich 90 mg thrombinfällbares Protein, Gesamteiweißgehalt der Lösung etwa 10 Gewichtsprozent ; etwa 20-30 Minuten vor der geplanten Verwendung aufgetaut). Man läßt das Gemisch in Zylindern aus rostfreiem Stahl (Innendurchmesser 6 mm, Höhe 10 mm) erstarren (1 ml für 3 Zylinder). Die gebildeten Gel-Zylinder werden anschließend aus den Formen ausgestoßen.

Beispiel G : Inhalations-Kapseln

5 kg Mono-Na-Salz des 3-p-Methoxybenzyliden-6-hydroxy-4'-methoxyflavanon-6-phosphats und 5 kg Lactose werden gemischt und das Gemisch in üblicher Weise in Kapseln gefüllt, so daß jede Kapsel 50 mg Wirkstoff enthält. Die Kapseln können mit Hilfe eines Inhalators appliziert werden.

**Patentansprüche**

1. Flavanonderivate der Formel I

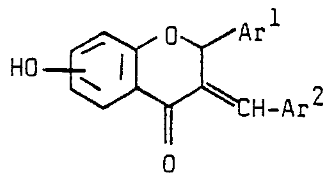

(I)

worin $Ar^1$ und $Ar^2$ jeweils unsubstituiertes oder ein- bis dreifach durch OH, Alkyl, Alkoxy, Acylamino, Halogen, COOAlkyl und/oder $NO_2$ und/oder durch eine Methylendioxygruppe substituiertes Phenyl bedeuten und die Alkyl-, Alkoxy- und Acylgruppen jeweils 1-7 C-Atome haben,
worin jedoch die HO-Gruppe nur dann in 6-Stellung steht, wenn mindestens einer der Reste $Ar^1$ bzw. $Ar^2$ substituiertes Phenyl bedeutet,
sowie ihre Phosphorsäureester und die Salze dieser Verbindungen.

2. a) Salze von Phosphorsäureestern der Verbindungen der Formel I nach Anspruch 1 mit Aminoglykosidantibiotika.

b) Gentamycinsalz des 3-p-Methoxybenzyliden-6-hydroxy-4'-methoxy-flavanon-6-phosphorsäureesters.

3. Verfahren zur Herstellung von Verbindungen nach Anspruch 1, dadurch gekennzeichnet, daß man ein Flavanon der Formel II

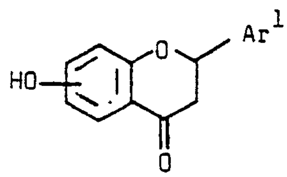

(II)

worin $Ar^1$ die bei Formel I angegebene Bedeutung hat mit einem Aldehyd der Formel III

$$O = CH—Ar^2 \qquad (III)$$

worin $Ar^2$ die bei Formel I angegebene Bedeutung hat umsetzt
oder daß man ein Chalkon der Formel IV

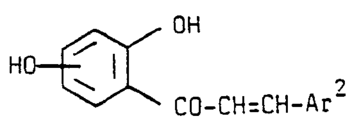

(IV)

worin $Ar^2$ die bei Formel I angegebene Bedeutung hat mit einem Aldehyd der Formel V

$$O = CH—Ar^1 \qquad (V)$$

worin $Ar^1$ die bei Formel I angegebene Bedeutung hat umsetzt
und/oder daß man ein Hydroxyflavanon der Formel I durch Behandeln mit einem phosphorylierenden Mittel in den entsprechenden Phosphorsäureester umwandelt
und/oder daß man eine Verbindung der Formel I beziehungsweise ihren Phosphorsäureester durch Behandeln mit einer Base in eines ihrer Salze überführt.

4. Verfahren zur Herstellung pharmazeutischer Zubereitungen, dadurch gekennzeichnet, daß man eine Verbindung der Formel I und/oder einen ihrer Phosphorsäureester und/oder ein physiologisch unbedenkliches Salz einer dieser Verbindungen zusammen mit mindestens einem festen, flüssigen oder

halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer Phosphorsäureester und/oder einem physiologisch unbedenklichen Salz einer dieser Verbindungen.

6. Verbindungen der Formel I, ihre Phosphorsäureester und/oder die physiologisch unbedenklichen Salze dieser Verbindungen zur Bekämpfung von Krankheiten.

7. Verwendung von Verbindungen der Formel I, ihrer Phosphorsäureester und/oder der physiologisch unbedenklichen Salze dieser Verbindungen zur Herstellung von pharmazeutischen Zubereitungen.

**Claims**

1. Flavanone derivatives of the formula I

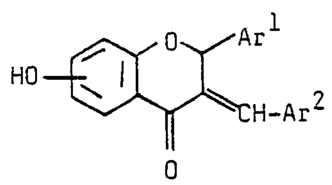

(I)

wherein $Ar^1$ and $Ar^2$ are each phenyl which is unsubstituted or monosubstituted to trisubstituted by OH, alkyl, alkoxy, acylamino, halogen, COOalkyl and/or $NO_2$ and/or substituted by a methylenedioxy group, and the alkyl, alkoxy and acyl groups each have 1-7 C atoms, but wherein the HO group is only in the 6-position if at least one of the radicals $Ar^1$ and $Ar^2$ is substituted phenyl, and phosphoric acid esters thereof and the salts of these compounds.

2. a) Salts of phosphoric acid esters of the compounds of the formula I according to Claim 1 with aminoglycoside antibiotics.

b) The gentamycin salt of 3-p-methoxybenzylidene-6-hydroxy-4'-methoxyflavanone-6-phosphoric acid ester.

3. Process for the preparation of compounds according to Claim 1, characterised in that a flavanone of the formula II

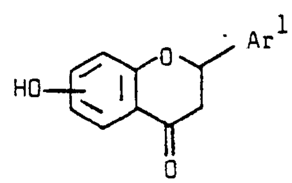

(II)

wherein $Ar^1$ has the meaning indicated in formula I is reacted with an aldehyde of the formula III

$$O = CH—Ar^2 \qquad (III)$$

wherein $Ar^2$ has the meaning indicated in formula I, or a chalcone of the formula IV

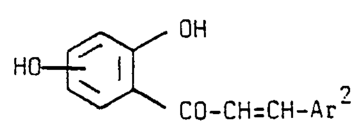

(IV)

wherein $Ar^2$ has the meaning indicated in formula I is reacted with an aldehyde of the formula V

$$O = CH—Ar^1 \qquad (V)$$

wherein $Ar^1$ has the meaning indicated in formula VI, and/or a hydroxyflavanone of the formula I is converted into the corresponding phosphoric acid ester by treatment with a phosphorylating agent, and/or a compound of the formula I or its phosphoric acid ester is converted into one of its salts by treatment with a base.

4. Process for the preparation of pharmaceutical formulations, characterised in that a compound of the formula I and/or one of its phosphoric acid esters and/or a physiologically acceptable salt of one of these compounds is brought into a suitable dosage form together with at least one solid, liquid or semiliquid excipient or auxiliary and, if appropriate, in combination with one or more further active compound(s).

5. Pharmaceutical formulation characterised in that it contains at least one compound of the formula I and/or one of its phosphoric acid esters and/or a physiologically acceptable salt of one of these compounds.

6. Compounds of the formula I, their phosphoric acid esters and/or the physiologically acceptable salts of these compounds for combating diseases.

7. The use of compounds of the formula I, their phosphoric acid esters and/or the physiologically acceptable salts of these compounds for the manufacture of pharmaceutical formulations.

**Revendications**

1. Dérivés de flavanone de formule I :

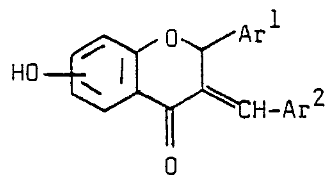

(I)

dans laquelle $Ar^1$ et $Ar^2$ représentent chacun un groupe phényle non substitué ou substitué une à trois fois par un groupe OH, un groupe alkyle, un groupe alcoxy, un groupe acylamino, un atome d'halogène, un groupe COOalkyle et/ou un groupe $NO_2$ et/ou par un groupe méthylène-dioxy,
les groupes alkyle, alcoxy et acyle contenant chacun 1 à 7 atomes de carbone, cependant que le groupe OH n'occupe que la position 6 lorsqu'au moins un des radicaux $Ar^1$ et $Ar^2$ représente un groupe phényle substitué,
de même que leurs esters d'acide phosphorique et les sels de ces composés.

2. a) Sels d'esters d'acide phosphorique des composés de formule I selon la revendication 1 avec des antibiotiques d'aminoglycosides,

b) sels de gentamycine de l'ester de l'acide 6-phosphorique de la 3-p-méthoxybenzylidène-6-hydroxy-4'-méthoxy-flavanone.

3. Procédé de préparation de composés selon la revendication 1, caractérisé en ce qu'on fait réagir une flavanone de formule II :

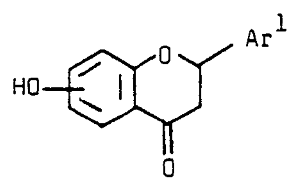

(II)

dans laquelle $Ar^1$ a la signification indiquée pour la formule I, avec un aldéhyde de formule III :

$$O = CH—Ar^2 \quad (III)$$

dans laquelle $Ar^2$ a la signification indiquée pour la formule I, ou en ce qu'on fait réagir une chalcone de formule IV :

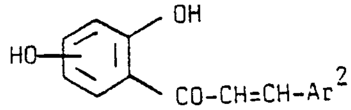

(IV)

dans laquelle $Ar^2$ a la signification indiquée pour la formule I, avec un aldéhyde de formule V :

$$O = CH—Ar^1 \quad (V)$$

dans laquelle $Ar^1$ a la signification indiquée pour la formule I, et/ou on convertit une hydroxyflavanone de formule I en son ester d'acide phosphorique
correspondant par traitement avec un agent de phosphorylation et/ou on transforme un composé de formule I ou son ester d'acide phosphorique en un de ses sels par traitement avec une base.

4. Procédé en vue d'obtenir des préparations pharmaceutiques, caractérisé en ce qu'on met, sous une forme posologique appropriée, un composé de formule I et/ou un de ses esters d'acide phosphorique et/ou un sel physiologiquement inoffensif d'un de ces composés conjointement avec au moins une substance auxiliaire ou une substance support solide, liquide ou semi-liquide et éventuellement en combinaison avec une ou plusieurs autres substances actives.

5. Préparation pharmaceutique, caractérisée en ce qu'elle contient au moins un composé de formule I et/ou un de ses esters d'acide phosphorique et/ou un sel physiologiquement inoffensif d'un de ces composés.

6. Composés de formule I, leurs esters d'acide phosphorique et/ou les sels physiologiquement inoffensifs de ces composés en vue de combattre les maladies.

7. Utilisation de composés de formule I, de leurs esters d'acide phosphorique et/ou des sels physiologiquement inoffensifs de ces composés en vue d'obtenir des préparations pharmaceutiques.